# FASCICULE DE BREVET EUROPEEN

(11) **EP 0 652 010 B1**
(45) Date de publication et mention de la délivrance du brevet: **07.01.1998**
(21) Numéro de dépôt: 94116721.5
(22) Date de dépôt: 30.01.1991
(51) Int. Cl.: A61K 31/665, A61K 31/355, A61K 9/127, A61K 7/00, A61K 7/48

(54) **Utilisation d'un phosphate d'alpha-tocophérol, ou d'un de ses dérivés pour la préparation d'une composition cosmétique, dermatologique ou pharmaceutique, à activité anti-radicalaire ; composition ainsi obtenue**
Verwendung von Alphatocopherol phosphate oder eines Derivates davon zur Herstellung kosmetischer, dermatologischer oder pharmazeutischer Präparate mit anti-radikaler Aktivität und Zusammensetzungen
Use of alpha-tocopherol phosphate or a derivative for the preparation of cosmetic, dermatalogical or pharmaceutical compositions with anti radical activity and composition thereby obtained

(30) Priorité: 31.01.1990 FR 9001143
(43) Date de publication de la demande: 10.05.1995
(62) Demande divisionnaire de: 91903316.7
(73) Titulaire: LVMH RECHERCHE, 92752 Nanterre (FR)
(72) Inventeur: Meybeck, Alain, F-92400 Courbevoie (FR); Bonte, Frédéric, F-92400 Courbevoie (FR); Marechal, Christian, F-75003 Paris (FR)
(74) Mandataire: Portal, Gérard

(56) Documents cités:
- EP-A- 0 226 753
- EP-A- 0 288 969
- EP-A- 0 332 478
- WO-A-87/02219
- WO-A-89/03689
- CHEMICAL ABSTRACTS, vol. 96, no. 21, 24 Mai 1982, Columbus, Ohio, US; abstract no. 179855q, F.KUZUDANI 'Effect of dl-alpha-tocopherol phosphoric acid ester on platelets' page 594 ; & YAKUBUTSU RYOHO, vol.14, no.3, 1981 pages 167 - 70
- CHEMICAL ABSTRACTS, vol. 85, no. 13, 27 Septembre 1976, Columbus, Ohio, US; abstract no. 87133x, SAKAI ET AL 'Activation of cyclic AMP phosphodiesterase by a new vitamin E derivative' page 23 ; & J. CYCLIC NUCLEOTIDE RES., vol.2, no.3, 1976 pages 163 - 70
- CHEMICAL ABSTRACTS, vol. 107, no. 10, 7 Septembre 1987, Columbus, Ohio, US; abstract no. 83908d, page 390 ; & RO-A-89 761 (INSTITUTUL ONCOLOGIC, BCURESTI) 30 Juillet 1986
- CHEMICAL ABSTRACTS, vol. 101, no. 6, 6 Août 1984, Columbus, Ohio, US; abstract no. 43589v, page 324 ; & JP-A-59 044 375 (SENJU PHARMACEUTICAL CO. LTD.) 12 Mars 1984
- PATENT ABSTRACTS OF JAPAN vol. 11, no. 371 (C-462) (2813) 3 Décembre 1987 & JP-A-62 145 019 (SENJYU SEIYAKU K.K.) 29 Juin 1987
- NATURWISSENSCHAFTEN, vol.47, 1960 page 17 MARUZZELLA, J.C. ET AL 'The action of anticoagulants on microorganisms'
- HELV. CHIM. ACTA, vol.23, 1940 pages 1137 - 1138 KARRER, P. ET AL 'd,l-alpha-tocopherol-phosphosäure-ester'
- CAN. J. BIOCHEM. BIOPHYS., vol.37, 1959 pages 501 - 505 LOWENTHAL, J. ET AL 'The inhibition of blood clotting by phosphate esters of vitamins K and E'

## Description

La présente invention concerne généralement l'utilisation d'un phosphate d'α-tocophérol, ou de l'un de ses esters, ou d'un sel de ces composés pour la préparation de compositions pharmaceutiques, cosmétiques ou dermatologiques pour la prévention ou le traitement des effets nocifs des radicaux libres, ainsi que des compositions pharmaceutiques, cosmétiques ou dermatologiques pour la prévention ou le traitement des effets nocifs des radicaux libres, l'incorporant.

On sait que la vitamine E a comme nom commun notamment l'α-tocophérol (voir Meck Index,10^{ème} édition, Référence 9 832, page 1 437).

L'α-tocophérol se trouve à l'état naturel dans de nombreuses plantes, habituellement avec d'autres composés tels que le β-tocophérol et l'α-tocophérol.

On sait également que l'α-tocophérol se présente sous les deux formes dl et d.

L'α-tocophérol est essentiellement utilisé pour lutter contre les déficiences en vitamine E, ou comme facteur nutritionnel, notamment pour lutter contre la dégénéréscence musculaire.

Il est également utilisé comme anti-oxydant, mais à des doses très spécifiques.

On a également décrit des esters d'α-tocophérol, et en particulier le succinate, le nicotinate ou l'acétate (Merck Index, 10^{ème} édition, Références 9 832, 9 833, page 1 437). La synthèse de l'acétate d'α-tocophérol est également décrite dans le document US-2 723 278, celle d'autres esters est décrite dans le document J. Amer. Chem. Soc. (1943) 65, 918-924.

Le phosphate de dl-α-tocophérol est également connu, (voir P. KARRER et al., Helv. Chim. Acta, (1940) 23 1137-8) ainsi que son action sur le métabolisme musculaire (voir J. Biol. Chem. 1942, 146, pages 309-321). Un autre document décrit le rôle biologique d'anti-oxydant sur du tissu cérébral (Biol. anti-oxydants Trans., 1 st Conf., 1946, pages 61-62). Il a également été décrit une action anticoagulante par une action sur la polymérisation de la fibrine (Can. J. Biochem. and Physiol. 1959, 37, pages 501-505). Une action anti-microbienne in vitro sur B. Subtilis et S. Aureus a également été décrite (Naturwissenchaften 1960, 47, page 17).

Par ailleurs, le document DE-A-3 416 209 décrit l'usage de crèmes à la vitamine E pour traiter et prévenir les processus inflammatoires. Par contre, Berkenkopf et Lutsky ont décrit que l'injection de la vitamine E chez le rat a provoqué une inflammation chronique localisée (Agents Actions 1979, 9, (4), 350-357).

Ainsi, l'action de la vitamine E sur l'inflammation est controversée.

Il a maintenant été découvert, de manière tout à fait surprenante et inattendue, que le phosphate d'α-tocophérol, notamment sous sa forme dl ou d, ou l'un de ses esters, de formule générale : 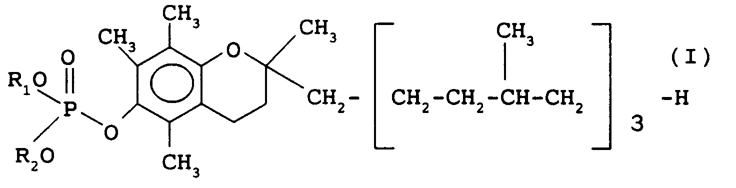 dans laquelle :
R₁ représente un atome d'hydrogène, un radical alkyle ayant de 1 à 4 atomes de carbone, tel qu'en particulier le radical méthyle ou éthyle, ou un radical α-tocophéryle;
R₂ représente un atome d'hydrogène, un radical alkyle ayant de 1 à 4 atomes de carbone, tel qu'en particulier un radical méthyle ou éthyle, ou bien R₂O représente une chaîne oxyéthylénée, de formule 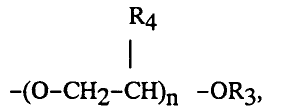 dans laquelle R₃ et R₄ réprésentent indépendamment un atome d'hydrogène ou un radical méthyle, et n représente un nombre entier supérieur ou égal à 1 ;
   ou l'un de ses sels,
   peut être utilisé pour la préparation d'une composition pharmaceutique, dermatologique ou cosmétique, destinée à la prévention ou au traitement des effets nocifs des radicaux libres, ou comme agent cosmétique anti-radicaux libres.

Ainsi, la présente invention a pour but de résoudre le nouveau problème technique consistant en la fourniture d'une substance active présentant une bonne activité préventive ou curative des effets nocifs des radicaux libres, en particulier par voie topique ou générale, en constituant ainsi un ingrédient actif précieux pour la préparation de compositions cosmétiques, dermatologiques ou pharmaceutiques.

La présente invention résout ce nouveau problème technique de manière satisfaisante, selon une solution particulièrement simple, utilisable à l'échelle industrielle.

Ainsi, selon le premier aspect, la présente invention couvre l'utilisation d'un phosphate d'α-tocophérol, notamment sous sa forme dl ou d ou l'un de ses esters, de formule générale : 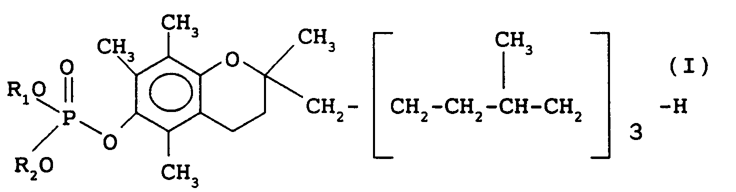 dans laquelle :
R₁ représente un atome d'hydrogène, un radical alkyle ayant de 1 à 4 atomes de carbone, tel qu'en particulier le radical méthyle ou éthyle, ou un radical α-tocophéryle ;
R₂ représente un atome d'hydrogène, un radical alkyle ayant de 1 à 4 atomes de carbone, tel qu'en particulier un radical méthyle ou éthyle, ou bien R₂O représente une chaîne oxyéthylénée, de formule 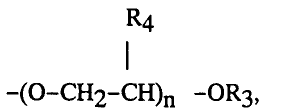 dans laquelle R₃ et R₄ réprésentent indépendamment un atome d'hydrogène ou un radical méthyl, et n représente un nombre entier supérieur ou égal à 1 ;
   ou l'un de ses sels,
   pour la préparation d'une composition pharmaceutique, dermatologique ou cosmétique, destinée à la prévention ou au traitement des effets nocifs des radicaux libres,
   ou comme agent cosmétique anti-radicaux libres.

Ainsi, les produits utilisés conformément à la présente invention sont des phosphates d'α-tocophérol ou leurs esters, ces produits pouvant se présenter sous forme de sels cosmétiquement, dermatologiquement ou pharmaceutiquement acceptables, tels que par exemple des sels de métaux alcalins, notamment de sodium (sel monosodique ou disodique), ou alcalino-terreux, notamment de magnésium, ou encore des sels d'ammonium ou d'amines primaires, secondaires ou tertiaires tels qu'en particulier la diéthylamine, la diéthanolamine, la triéthylamine ou la triéthanolamine.

Dans la formule (I), les radicaux alkyles peuvent être à chaîne droite ou ramifiée.

Un radical alkyle ayant de 1 à 4 atomes de carbone est par exemple méthyle, éthyle, propyle, isopropyle, butyle, de préférence méthyle ou éthyle.

Par radical α-tocophéryle, on entend désigner le radical : 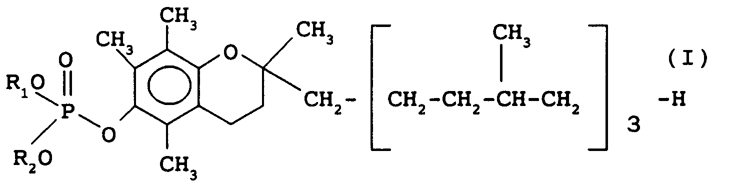 lorsque R₂O représente une chaîne oxyéthylénée, n sera généralement supérieur ou égal à 1, par exemple compris entre 2 et 50, de préférence entre 2 et 25 et en particulier égal à 2 ou 5.

Suivant un autre mode de réalisation avantageux, conforme à l'invention, on utilise un composé de formule I, tel que précédemment défini, de préférence à l'état de sel, sous forme de petites vésicules de type liposome obtenues par dispersion dudit composé ou dudit sel dans l'eau ou dans un milieu aqueux, tel qu'une solution tampon, notamment au moyen d'une agitation mécanique suivie d'une homogénéisation, par exemple à l'aide d'ultra-sons ou d'un homogénéiseur sous pression.

De préférence, on règle la taille de ces vésicules à une valeur comprise environ entre 6.10⁻² µm et 2 µm, en modifiant les paramètres de l'homogénéisation tels que l'énergie et la durée.

Suivant une variante avantageuse du précédent mode de réalisation, le milieu aqueux précité contient un agent biologiquement actif, ledit agent étant encapsulé au moins en partie après dispersion dans les vésicules précitées.

De préférence, l'agent actif précité est une substance anti-allergique, telle qu'un extrait de Scutellaria, comme par exemple un extrait de racine de Scutellaria Baicalensis Georgi décrit dans le document FR-A- 2 628 317, ou une substance anti-inflammatoire.

Selon un mode de réalisation avantageux de l'utilisation conforme à l'invention, la concentration en poids du composé de formule I précité, ou de l'un de ses sels, est comprise entre 0,001 et 10%, de préférence entre 0,01 % et 1 %, et de préférence encore entre 0,05 et 0,5 %, par rapport au poids total de la composition.

Selon un mode de réalisation actuellement préféré, le composé de formule (I) précitée est le phosphate de dl-α-tocophérol. Les sels préférés sont les sels monosodiques et le sel disodique.

Les composés utilisés conformément à l'invention sont généralement disponibles dans le commerce et peuvent être notamment préparés en suivant des processus décrits dans la littérature, par exemple dans : Chem. Pharm. Bull, (1971), 19, (4), pages 687 à 695 ; Khim.-Pharm. Zh (1983), 17, (7), pages 840 à 844 ; Khim.-Pharm. Zh (1985), 19, (1), pages 75 à 77 ou encore dans les brevets US-2 457 932 ou JP-54-54 978.

La concentration en ingrédient actifs dans ces compositions cosmétiques ou dermatologiques est telle que décrite précédemment pour l'utilisation.

Les compositions selon l'invention peuvent être formulées selon toute forme acceptable pour leur emploi en cosmétologie, dermatologie ou phamacie. Il peut en particulier s'agir de crème préventive et curative des allergies cutanées, de crème anti-allergique calmante, d'huile calmante anti-allergique, de lotion anti-allergique préventive ou curative, de lotion alcoolique après-rasage pour calmer les irritations de la peau, de crème hypo-allergénique, de solution colloïdale anti-asthmatique.

Les compositions selon l'invention peuvent être également formulées sous forme de compositions de maquillage telles que fond de teint, rouge à lèvres, mascara, poudre teintée.

L'incorporation du composé de formule I ou de l'un de ses sels dans ladite composition cosmétique, dermatologique ou pharmaceutique peut être effectuée selon différentes manières accessibles à l'homme de l'art, selon le type de formule désirée.

Suivant un mode avantageux de mise en oeuvre desdits procédés de fabrication, lorsque la composition comprend une phase aqueuse, le composé de formule (I) précité est dispersé, de préférence à l'état de sel tel que déjà défini, préalablement dans de l'eau ou dans ladite phase aqueuse pour former de petites vésicules, et la dispersion ainsi obtenue est ensuite mélangée aux autres constituants éventuels de la composition.

Selon un autre aspect, la présente invention couvre un procédé de prévention ou de traitement, cosmétique, des effets nocifs des radicaux libres, caractérisé en ce qu'il comprend l'application d'une quantité efficace d'au moins un composé de formule (I) ou l'un de ses esters ou sels tels que précédemment définis, incorporé dans un excipient, véhicule ou support cosmétiquement, dermatologiquement ou pharmaceutiquement acceptable.

L'invention sera maintenant illustrée en détail à l'aide des exemples de réalisation suivants.

Dans les exemples, les pourcentages sont donnés en poids sauf indication contraire.

### Exemple 1

### a) Préparation d'une suspension de phosphate monosodique de dl-α-tocophérol

On pèse 0,8 g de poudre de phosphate disodique de dl-α-tocophérol obtenu suivant la méthode décrite par P. KARRER (Helv. Chim. Acta, (1940) 23, 1137-8).

On verse cette poudre dans 96,2 g d'eau bidistillée, sous agitation que l'on poursuit pendant environ 2 heures.

On effectue ensuite une homogénéisation par ultrasons pendant 10 min à 150 W jusqu'à obtention d'une suspension limpide, donnant lieu à l'obtention de vésicules de type liposome de phosphate de tocophérol disodique.

Dans le cas de volumes plus importants, on peut avantageusement utiliser un homogénéiseur sous pression, par exemple de type Manton-Gaulin® à la pression de 500 bars environ.

On abaisse ensuite le pH jusqu'à 7 sous agitation avec ajout d'environ 3 ml d'HCl 0,5 N, puis on ajuste le pH à 6,5 sous agitation avec HCl 0,1 N. A ce pH, le phosphate de tocophérol est alors sous forme de sel monosodique.

On peut déterminer la dimension des vésicules de phosphate monosodique d'α-tocophérol ainsi obtenue par exemple au moyen d'un Autosizer 2C de la société MALVERN. Dans cet exemple, la taille moyenne mesurée est de l'ordre de 100 nm.

On observera également que l'on peut réaliser diverses dilutions en modifiant la quantité de composés ajoutés au départ ou en modifiant le volume de la solution de dispersion, ce qui constitue un procédé aisé de préparation de diverses concentrations en principe actif.

Dans l'exemple décrit, on a obtenu environ 100 g de suspension contenant environ 0,8 % de phosphate monosodique de dl-α-tocophérol sous forme de vésicules de type liposome, de taille sensiblement homogènes.

### b) Préparation d'une composition gélifiée de phosphate monosodique d'α-tocophérol

La suspension homogénéisée obtenue précédemment peut être gélifiée par mélange avec un gel, tel qu'un gel de polymère vinylique, en particulier commercialisé sous la dénomination commerciale Carbopol® 940.

D'une façon connue en soi, on peut préparer ce gel par exemple en dispersant 1 g de Carbopol® 940 dans 99 g d'eau en présence d'un conservateur, puis, après gonflement, en neutralisant à pH 7,5, avec par exemple de la triéthanolamine.

Aux 100 g de suspension homogénéisée obtenue précédemment, on ajoute 100 g de ce gel, pour obtenir une composition gélifiée, dont la concentration en phosphate monosodique d'α-tocophérol est d'environ 0,4 %.

Des compositions gélifiées de concentration variées en phosphate d'α-tocophérol peuvent être obtenues en suivant le procédé indiqué ci-dessus.

### Exemple 2

### Mise en évidence de l'activité anti-radicalaire des compositions conformes à l'invention

### Etude de l'activité anti-radicalaire

Pour cette étude, on procède selon le protocole décrit par : M.S. NOEL-HUDSON, C. de BELILOVSKI, N. PETIT, A. LINDENBAUM, J. WEPIERRE dans TOXIC. in vitro, 1989 3 (2) 103-109.

On effectue des essais sur des cultures de kératinocytes humains. La solution mère du produit à tester selon l'invention est une solution aqueuse à 0,1 % de phosphate disodique de dl-α-tocophérol. Cette solution est utilisée à différentes dilutions dans du milieu de culture MCDB153 (Irvine®) supplémenté en éthanolamine, phosphoéthanolamine, cortisone, insuline, calcium (0,1 mM), de manière à obtenir les concentrations suivantes en sel de phosphate de dl-α-tocophérol :
10⁻³ %, 5.10⁻⁴ %, 10⁻⁴ % et 5.10⁻⁵ %.

La dilution à tester est mise en contact avec les cellules en culture pendant 48 heures, juste après l'ensemencement.

Le milieu de culture est ensuite éliminé et les cellules sont rincées au tampon phosphate. Le système hypoxanthine-xanthine oxydase (HX-XO), générateur de radicaux libres, est alors appliqué pendant 2 heures 30. Après un nouveau rinçage au tampon phosphate, la cytotoxicité est déterminée par la méthode dite au rouge neutre (Borenfreund et Puerner, 1985).

Les valeurs figurant au tableau I représentent la viabilité cellulaire exprimée en pourcentage de cellules vivantes par rapport au nombre total de cellules dans la culture considérée.

**TABLEAU I**

| | Concentrations du produit à tester | | | | |
|---|---|---|---|---|---|
| | 0 % | 10⁻³ % | 5.10⁻⁴ % | 10⁻⁴ % | 5.10⁻⁵ % |
| Cultures témoin | 100 | 66,40 | 87,92 | 94,28 | 100 |
| Cultures traitées (HX-XO) | 10,57 | 39,59 | 32,79 | 12,19 | 9,28 |

On observe que le pourcentage de viabilité cellulaire est très nettement amélioré dans les cultures ayant été en contact avec le produit selon l'invention, préalablement au traitement par le xystème HX-XO, par rapport à celle de la culture n'ayant reçu préalablement aucun produit.

Ceci montre de façon très nette, l'activité protectrice préventive du produit selon l'invention vis-à-à-vis de l'action cytotoxique des radicaux libres, tels que ceux produits par le système hypoxanthine-xanthine oxydase.

### Exemples de formules pharmaceutiques ou cosmétiques contenant du phosphate de vitamine E

### Exemple 3

Crème préventive et curative des allergies cutanées. Composition :

| | | |
|---|---|---|
| A - | Cera bellina | 5,00 g |
| | Silicone 200 | 1,50 g |
| | Squalane | 5,00 g |
| | Myglyol 812 | 5,00 g |
| | Nylon 12 Sp 500 | 3,00 g |
| | BHT | 0,05 g |
| B - | eau déminéralisée | 49,56 g |
| | EDTA | 0,10 g |
| | Propylène glycol | 4,00 g |
| | Carbopol® 1342 | 0,45 g |
| | Triéthanolamine | 0,54 g |
| | Dispersion de phosphate de dl-α-tocophérol monosodique à 0,4 %, pH 6,6 | 25,00 g |
| C - | Germaben II® | 0,80 g |

Mode opératoire : On chauffe le mélange A sous agitation, afin d'obtenir un mélange homogène. Pour préparer le mélange B, on disperse le Carbopol® 1342 dans la solution aqueuse contenant l'EDTA et le propylène glycol dans 49,56 g d'eau distillée, et on neutralise avec la triéthanolamine. On ajoute ensuite la dispersion à 0,4 % (non gélifiée) de phosphate de dl-α-tocophérol obtenu selon l'exemple 1.

On porte ensuite le mélange B à 75°C et on le maintient à cette température sous agitation pendant que l'on y ajoute le mélange A. On laisse refroidir à 45°C, puis on ajoute le Germaben II®, et on laisse refroidir encore, sous agitation, jusqu'à température ambiante.

On obtient ainsi une crème.

### Exemple 4

Crème anti-allergique calmante

### Composition :

| | | |
|---|---|---|
| A - | Lécithine de soja | 2,00 g |
| | Cosbiol® | 8,50 g |
| B - | Eau déminéralisée | 58,85 g |
| | EDTA | 0,10 g |
| | Glycérine | 4,00 g |
| | Carbopol®940 | 0,35 g |
| | Triéthanolamine | 0,40 g |
| | Germaben II® | 0,80 g |
| C - | Dispersion phosphate d'α-tocophérol monosodique à 0,4 %, pH 6,6 | 25,00 g |

Mode opératoire : On chauffe sous agitation le Cosbiol® et la lécithine jusqu'à dissolution complète, et on laisse refroidir à température ambiante. Le mélange B est obtenu en dispersant le Carbopol® 940 dans le mélange eau+EDTA+glycérine. On neutralise le tout avec la triéthanolamine, puis on ajoute le Germaben II®.

On verse ensuite sous agitation le mélange A sur le mélange B. On homogénéise, puis on ajoute la dispersion obtenue comme à l'exemple 1. On homogénéise encore et on obtient ainsi une crème utilisable matin et soir pour calmer les réactions allergiques cutanées en applications locales.

### Exemple 5

### Huite calmante anti-allergique

On dissout 0,1 g de poudre de phosphate d'α-tocophérol disodique dans 99,9 g de trioctyle citrate sous agitation magnétique à 70°C pendant 8 h.

La solution huileuse ainsi obtenue peut être utilisée en applications locales, comme la crème de l'exemple 4.

### Exemple 6

Letion alcoolique après-rasage

### Composition :

| | |
|---|---|
| Phosphate d'α-tocophérol disodique | 0,2 g |
| Ethanol | 40 g |
| Propylène glycol | 0,5 g |
| Pantothénol | 0,1 g |
| Excipient aqueux parfumé q.s.p. | 100 g |

Préparation : On dissout séparément le phosphate de tocophérol disodique dans l'alcool absolu d'une part, et les autres constituants dans l'eau d'autre part. On mélange les deux solutions obtenues et on homogénéise le tout au moyen d'ultrasons.

Cette lotion permet de calmer les irritations dues au rasage, appelées couramment "feu du rasage".

### Exemple 7

Lotion anti-allergique préventive ou curative.

### Composition :

| | |
|---|---|
| Dispersion de phosphate d'α-tocophérol monosodique à 4 % | 25,00 g |
| Ethanol | 10,00 g |
| Propylène glycol | 5,00 g |
| Excipient aqueux q.s.p. | 100,00 g |

La dispersion à 4 % de phosphate d'α-tocophérol est préparée comme à l'exemple 1, si ce n'est qu'elle est plus concentrée en phosphate d'α-tocophérol monosodique.

Les constituants de la formule ci-dessus sont mélangés entre eux et homogénéisés au moyen d'ultrasons.

### Exemple 8

Solution colloïdale anti-asthmatique

### Composition

| | |
|---|---|
| Dispersion à 4 % de phosphate d'α-tocophérol monosodique | 12,50 g |
| Excipient aqueux tamponné + conservateur q.s.p. | 100,00 g |

La dispersion de phosphate de tocophérol monosodique est préparée comme à l'exemple 1. On obtient après homogénéisation aux ultrasons une solution colloïdale que l'on incorpore ensuite à l'excipient tamponné.

Cette solution peut être utilisée en pulvérisation dans les voies respiratoires supérieures, notamment pour calmer les toux asthamatiformes.

### Exemple 9

Solution colloïdale pour les techniques de réanimation.

### Composition

| | |
|---|---|
| Dispersion à 4 % de phosphate d'α-tocophérol monosodique | 7,50 g |
| Excipient aqueux tamponné + conservateur q.s.p. | 100,00 g |

Cette composition est préparée comme à l'exemple précédent.

Elle peut être utilisée pour combattre les effets toxiques des radicaux superoxydes qui se forment lors de la mise en oeuvre des techniques de réanimation avec l'oxygène. Elle est alors administrée en instillation intratrachéale, en même temps que l'administration du mélange gazeux.

### Exemple 10

Fond de teint anti-allergique.

### Composition :

| | |
|---|---|
| Phosphate de dl-α-tocophérol disodique | 0,5 g |
| Emulsion pour fond de teint | 99,5 g |

On prépare cette composition en incorporant à la phase aqueuse de l'émulsion, le phosphate de tocophérol disodique préalablement dispersé dans l'eau. On procède ensuite de manière classique pour réaliser l'émulsion.

Ce fond de teint minimise les risques de manifestations allergiques dûs à une matière première ou à une substance allergène venant en contact de la peau.

## Revendications

1. Utilisation d'un phosphate d'α-tocophérol, notamment sous sa forme dl ou d, ou l'un de ses esters, de formule générale : 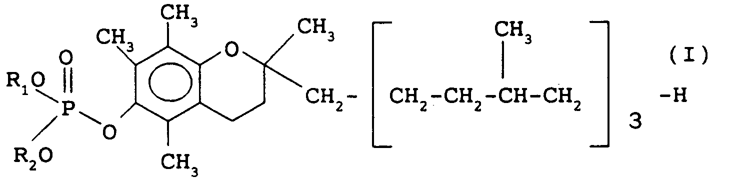 dans laquelle :
R₁ représente un atome d'hydrogène, un radical alkyle ayant de 1 à 4 atomes de carbone, tel qu'en particulier le radical méthyle ou éthyle, ou un radical α-tocophéryle ;
R₂ représente un atome d'hydrogène, un radical alkyle ayant de 1 à 4 atomes de carbone, tel qu'en particulier un radical méthyle ou éthyle, ou bien R₂O représente une chaîne oxyéthylénée, de formule 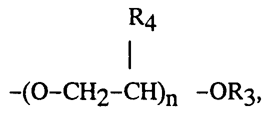 dans laquelle R₃ et R₄ réprésentent indépendamment un atome d'hydrogène ou un radical méthyle, et n représente un nombre entier supérieur ou égal à 1 ;
ou l'un de ses sels,
pour la préparation d'une composition pharmaceutique, dermatologique ou cosmétique, destinée à la prévention ou au traitement des effets nocifs des radicaux libres,
ou comme agent cosmétique anti-radicaux libres.

2. Utilisation selon la revendication 1 d'un composé de formule (I), tel que précédemment défini, de préférence à l'état de sel, sous forme de petites vésicules de type liposome obtenues par dispersion dudit composé ou dudit sel dans l'eau ou dans un milieu aqueux tel qu'une solution tampon.

3. Utilisation selon la revendication 2, caractérisée en ce que la taille des vésicules est comprise environ entre 6.10⁻² µm et 2 µm.

4. Utilisation selon l'une des revendications 2 ou 3, caractérisée en ce que le milieu aqueux précité contient un agent biologiquement actif, ledit agent étant encapsulé au moins en partie après dispersion dans les vésicules précitées.

5. Utilisation selon la revendication 4, caractérisée en ce que l'agent biologiquement actif est une substance anti-allergique, telle qu'un extrait de Scutellaria comme par exemple un extrait de racine de Scutellaria Baicalensis Georgi, ou une substance anti-inflammatoire.

6. Utilisation selon l'une des revendications 1 à 5, caractérisée en ce que la concentration en poids du composé de formule (I) précitée, ou de l'un de ses sels, est comprise entre 0,001 et 10 %, de préférence entre 0,01 % et 1 %, et de préférence encore entre 0,05 % et 0,5 %, par rapport au poids total de la composition.

7. Utilisation selon l'une des revendications 1 à 6, caractérisée en ce que le composé de formule (I) précitée est le phosphate de dl-α-tocophérol.

8. Utilisation selon l'une des revendications 1 à 7, caractérisée en ce que le sel du composé de formule (I) précitée est un sel monosodique ou le sel disodique.

9. Procédé de prévention ou de traitement cosmétique des effets nocifs des radicaux libres, caractérisé en ce qu'il comprend l'application d'une quantité efficace d'au moins un composé de formule (I) ou de l'un de ses esters ou sels, tels que définis à l'une quelconque des revendications 1 à 8, incorporé dans un excipient, véhicule ou support cosmétiquement acceptable.

## Claims

1. Use of an α-tocopherol phosphate, especially in its dl or d form, or an ester thereof, of the general formula 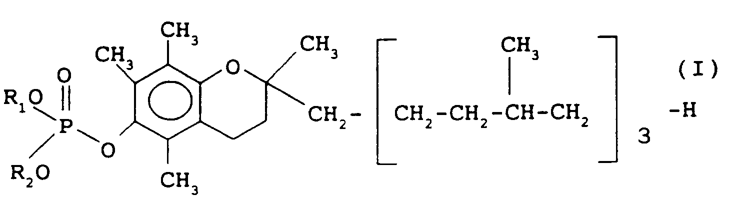 in which:
R₁ is a hydrogen atom, an alkyl radical having from 1 to 4 carbon atoms, such as the methyl or ethyl radical in particular, or an α-tocopheryl radical;
R₂ is a hydrogen atom, an alkyl radical having from 1 to 4 carbon atoms, such as a methyl or ethyl radical in particular, or else R₂O is an oxyethylenated chain of the formula 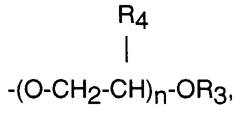 in which R₃ and R₄ independently are a hydrogen atom or a methyl radical and n is an integer greater than or equal to 1;
or a salt thereof,
for preparing a pharmaceutical, dermatological or cosmetic composition intended for the prevention or treatment of the harmful effects of the free radicals,
or as antifree radical cosmetic agent.

2. Use according to claim 1 of a compound of formula (I) as defined above, preferably as a salt, in the form of small liposome-type vesicles obtained by dispersing said compound or said salt in water or in an aqueous medium such as a buffer solution.

3. Use according to claim 2, characterized in that the size of the vesicles is between about 6.10⁻² µm and 2 µm.

4. Use according to one of claims 2 or 3, characterized in that the above-mentioned aqueous medium contains a biologically active agent, said agent being at least partially encapsulated after dispersion in the above-mentioned vesicles.

5. Use according to claim 4, characterized in that the biologically active agent is an antiallergic substance such as an extract of Scutellaria, for example an extract of the root of Scutellaria Baicalensis Georgi, or an antiinflammatory substance.

6. Use according to one of claims 1 to 5, characterized in that the concentration by weight of the compound of formula (I) mentioned above, or a salt thereof, is between 0.001 and 10%, preferably between 0.01% and 1% and particularly preferably between 0.05% and 0.5%, relative to the total weight of the composition.

7. Use according to one of claims 1 to 6, characterized in that the compound of formula (I) mentioned above is dl-α-tocopherol phosphate.

8. Use according to one of claims 1 to 7, characterized in that the salt of the compound of formula (I) mentioned above is a monosodium salt or the disodium salt.

9. Cosmetic method for preventing or for treating the harmful effects of the free radicals, characterized in that it comprises applying an effective amount of at least one compound of formula (I) or a salt thereof, as defined in any one of claims 1 to 8, incorporated in a cosmetically acceptable excipient, vehicle or carrier.

## Patentansprüche

1. Verwendung eines α-Tocopherolphosphats, insbesondere in seiner dl- oder d-Form, oder eines seiner Ester, der allgemeinen Formel: 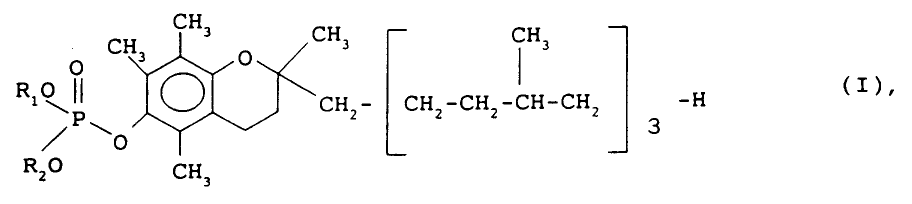 worin R₁ ein Wasserstoffatom, ein Alkylrest mit 1 bis 4 Kohlenstoffatomen, wie insbesondere ein Methyl- oder Ethylrest, oder ein α-Tocopherylrest ist; R₂ ein Wasserstoffatom, einen Alkylrest mit 1 bis 4 Kohlenstoffatomen, wie insbesondere einen Methyl- oder Ethylrest, darstellt, oder auch R₂O bedeutet: eine Oxyethylen-Kette der Formel 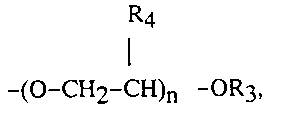 worin R₃ und R₄ unabhängig ein Wasserstoffatom oder einen Methylrest darstellen, und n eine ganze Zahl größer oder gleich 1 ist; oder eines seiner Salze, bei der Herstellung einer pharmazeutischen, dermatologischen oder kosmetischen Zusammensetzung zur Prävention oder zur Behandlung schädlicher Wirkungen freier Radikale, oder als kosmetisches Mittel gegen freie Radikale.

2. Verwendung nach Anspruch 1 einer Verbindung der Formel (I), wie vorstehend definiert, vorzugsweise als Salz, in Form kleiner Vesikel vom Liposomentyp, die durch die Dispersion der genannten Verbindung oder des genannten Salzes in Wasser oder in einem wässerigen Medium, wie einer Pufferlösung, erhalten werden.

3. Verwendung nach Anspruch 2, dadurch gekennzeichnet, daß die Größe der Vesikel etwa zwischen 6 x 10⁻² µm und 2 µm liegt.

4. Verwendung nach Anspruch 2 oder 3, dadurch gekennzeichnet, daß das vorgenannte wässerige Medium ein biologisch wirksames Mittel enthält, wobei das genannte Mittel zumindest teilweise nach der Dispersion in den vorgenannten Vesikeln eingekapselt wird.

5. Verwendung nach Anspruch 4, dadurch gekennzeichnet, daß das biologisch wirksame Mittel eine anti-allergische Substanz, wie ein Scutellaria-Extrakt, wie beispielsweise ein Wurzel-Extrakt von Scutellaria baicalensis georgi, oder eine entzündungshemmende Substanz ist.

6. Verwendung nach einem der Ansprüche 1 bis 5, dadurch gekennzeichnet, daß die Massekonzentration der vorgenannten Verbindung der Formel (I) oder eines ihrer Salze zwischen 0,001 und 10 %, vorzugsweise zwischen 0,01 % und 1 %, und vorzugsweise auch zwischen 0,05 % und 0,5 %, bezogen auf die Gesamtmasse der Zusammensetzung, beträgt.

7. Verwendung nach einem der Ansprüche 1 bis 6, dadurch gekennzeichnet, daß die vorgenannte Verbindung der Formel (I) dl-α-Tocopherolphosphat ist.

8. Verwendung nach einem der Ansprüche 1 bis 7, dadurch gekennzeichnet, daß das Salz der vorgenannten Verbindung der Formel (I) ein Mononatriumsalz oder ein Dinatriumsalz ist.

9. Kosmetisches Verfahren zur Vorbeugung oder Behandlung schädlicher Wirkungen freier Radikale, dadurch gekennzeichnet, daß es das Aufbringen einer wirksamen Menge zumindest einer Verbindung der Formel (I) oder eines ihrer Ester oder Salze, wie in einem der Ansprüche 1 bis 8 definiert, eingeschlossen in einem kosmetisch annehmbaren Exzipienten, Vehikel oder Träger, umfaßt.
